# EUROPEAN PATENT SPECIFICATION

(11) **EP 3 490 392 B1**
(45) Date of publication and mention of the grant of the patent: **23.11.2022**
(21) Application number: 17743017.0
(22) Date of filing: 21.07.2017
(51) Int. Cl.: A24F 47/00

(54) **AEROSOL-GENERATING SYSTEM COMPRISING A GEL CONTAINING CARTRIDGE AND A DEVICE FOR HEATING THE CARTRIDGE**
AEROSOLERZEUGUNGSSYSTEM MIT GELHALTIGER KARTUSCHE UND VORRICHTUNG ZUR ERWÄRMUNG DER KARTUSCHE
SYSTÈME DE GÉNÉRATION D'AÉROSOL COMPRENANT UN GEL CONTENANT UNE CARTOUCHE ET UN DISPOSITIF DE CHAUFFAGE DE LA CARTOUCHE

(30) Priority: 29.07.2016 EP 16181956
(43) Date of publication of application: 05.06.2019
(73) Proprietor: Philip Morris Products S.A., 2000 Neuchâtel (CH)
(72) Inventor: ZUBER, Gérard, CH 2000 Neuchâtel (CH); VOLLMER, Jean-Yves, CH 1421 Fontaines sur Grandson (CH)
(74) Representative: Ponder, William Anthony John
(86) International application number: PCT/EP2017/068549
(87) International publication number: WO 2018/019738

(56) References cited:
- WO-A1-95/27411
- WO-A1-2015/177254
- WO-A2-2015/040180
- US-A1- 2013 298 905
- US-A1- 2014 060 554
- US-A1- 2015 328 415
- US-A1- 2016 120 225
- DANIEL C ET AL: "On the definition of thermoreversible gels: the case of syndiotactic polystyrene", POLYMER, ELSEVIER SCIENCE PUBLISHERS B.V, GB, vol. 35, no. 19, 1 September 1994 (1994-09-01), pages 4243-4246, XP024117906, ISSN: 0032-3861, DOI: 10.1016/0032-3861(94)90604-1 [retrieved on 1994-09-01]

## Description

The present invention relates to an aerosol-generating system that heats an aerosol-forming substrate to generate an aerosol. In particular, the invention relates to an aerosol generating system that heats a gel to form an aerosol.

Aerosol-generating systems, such as e-cigarettes, that operate by heating a liquid formulation to generate an aerosol for inhalation by users are widely used. Typically they comprise device portion and a cartridge. In some systems, the device portion contains a power supply and control electronics and the cartridge contains a liquid reservoir holding the liquid formulation, a heater for vapourising the liquid formulation, and a wick that transports the liquid from the liquid reservoir to the heater. While this type of system has become popular, it does have disadvantages. One disadvantage is the potential for leakage of the liquid from the liquid reservoir both during transport and storage, and when the cartridge is connected to the device portion. The use of a wick to transport the liquid from the reservoir to the heater may add complexity to the system.

US2014/0060554 discloses an electronic smoking article comprising a series of microheaters for vaporization of an aerosol precursor composition. The aerosol precursor composition is comprised in a substrate which can be placed in a cavity in the electronic smoking article such that the top and bottom surfaces of the substrate are in contact with the microheaters. The aerosol precursor composition may have a variety of forms at ambient conditions. The aerosol precursor composition can be a liquid, a gel or a solid at ambient conditions.

In a first aspect of the invention, there is provided an aerosol-generating system comprising:
a device comprising a power supply and an electrical heater connected to the power supply; and
a substrate cartridge containing an aerosol-forming substrate in the form of a thermoreversible gel that is solid at room temperature;
wherein the substrate cartridge is configured to be inserted into or connected to the device prior to use and removed or disconnected from the device after use.

In this context, an aerosol-forming substrate is a material or mixture of materials capable of releasing volatile compounds that can form an aerosol. The provision of the aerosol-forming substrate in the form of a gel may be advantageous for storage and transport, or during use. By providing the aerosol-forming substrate in a gel, the risk of leakage from the device may be reduced. Replenishing of the device with aerosol forming substrate when depleted or exhausted may also be improved, for example by reducing the risk of leakage or spillage.

The provision of the heater within the device, not in the cartridge, allows for the production of relatively simple, cartridges compared with integration of the heater in the cartridge. Advantageously, the system does not comprise a transport mechanism for transporting the gel to the electrical heater. The contents of the substrate cartridge are advantageously heated *in situ* to generate a desired aerosol. In this context *in situ* means in the same position within substrate cartridge that the contents are held prior to use. There is no requirement for a capillary wick or pump. The electrical heater may be configured to heat the cartridge to generate a vapour within the cartridge from the gel.

The cartridge can be easily disposed of and replaced when the gel has been consumed.

The substrate container may contain other materials in addition to the gel.

The gel is solid at room temperature. "Solid" in this context means that the gel has a stable size and shape and does not flow. Room temperature in this context means 25 degrees Celsius.

The gel may comprise an aerosol-former. As used herein, the term "aerosol-former" refers to any suitable known compound or mixture of compounds that, in use, facilitates formation of a dense and stable aerosol. An aerosol-former is substantially resistant to thermal degradation at the operating temperature of the cartridge. Suitable aerosol-formers are well known in the art and include, but are not limited to: polyhydric alcohols, such as triethylene glycol, 1,3-butanediol and glycerine; esters of polyhydric alcohols, such as glycerol mono-, di- or triacetate; and aliphatic esters of mono-, di- or polycarboxylic acids, such as dimethyl dodecanedioate and dimethyl tetradecanedioate. Preferred aerosol formers are polyhydric alcohols or mixtures thereof, such as triethylene glycol, 1,3-butanediol and, most preferred, glycerine or polyethylene glycol..

The gel may comprise a gelling agent. Preferably, the gel comprises agar or agarose or sodium alginate. The gel may comprise Gellan gum.

The gel comprises a thermoreversible gel. This means that the gel will become fluid when heated to a melting temperature and will set into a gel again at a gelation temperature. The gelation temperature is preferably at or above room temperature and atmospheric pressure. Atmospheric pressure means a pressure of 1 atmosphere. The melting temperature is preferably higher than the gelation temperature. Preferably the melting temperature of the gel is above 50 degrees Celsius, or 60 degrees Celsius, or 70 degrees Celsius, and more preferably above 80 degrees Celsius. The melting temperature in this context means the temperature at which the gel is no longer solid and begins to flow. The gel may comprise a gelling agent. Preferably, the gel comprises agar or agarose or sodium alginate. The gel may comprise Gellan gum. The gel may comprise a mixture of materials. The gel may comprise water.

The gel may be provided as a single block or may be provided as a plurality of gel elements, for example beads or capsules. The use of beads or capsules may allow for simple refilling of the first (or second) chamber by the end user. The use of capsules or beads may also allow a user to see when a cartridge has already been used because gel will not form the same capsules or beads on gelation after heating and subsequent cooling.

The gel may comprise nicotine or a tobacco product or another target compound for delivery to a user. When the resulting aerosol is to contain nicotine, it is advantageous for the nicotine to be contained in the gel or in another solid form in the substrate container rather than in a liquid. The nicotine can be included in the gel with an aerosol-former. Nicotine is irritating to the skin and can be toxic. Preventing any possible leakage of nicotine by locking the nicotine into a gel at room temperature is therefore desirable.

Flavour compounds may be contained in the second chamber in a gel. Alternatively or in addition, flavour compound may be provided in another form. For example, the second chamber may contain a solid tobacco material that releases flavour compounds when heated. The second chamber may contain, for example, one or more of: powder, granules, pellets, shreds, spaghettis, strips or sheets containing one or more of: herb leaf, tobacco leaf, fragments of tobacco ribs, reconstituted tobacco, homogenised tobacco, extruded tobacco and expanded tobacco. The solid tobacco material in the second chamber may be in loose form. The tobacco may be contained in a gel or liquid. The second chamber may contain additional tobacco or non-tobacco volatile flavour compounds, to be released upon heating.

When agar is used as the gelling agent, the gel preferably comprises between 0.5 and 5% by weight (and more preferably between 0.8 and1% by weight) agar. The gel may further comprise between 0.1 and 2% by weight nicotine. The gel may further comprise between 30% and 90% by weight (and more preferably between 70 and 90% by weight) glycerin. A remainder of the gel may comprise water and any flavourings.

When Gellan gum is used as the gelling agent, the gel preferably comprises between 0.5 and 5% by weight Gellan gum. The gel may further comprise between 0.1 and 2% by weight nicotine. The gel may further comprise between 30% and 99.4% by weight gylcerin. A remainder of the gel may comprise water and any flavourings.

In one embodiment, the gel comprises 2% by weight nicotine, 70% by weight glycerol, 27% by weight water and 1% by weight agar. In another embodiment, the gel comprises 65% by weight glycerol, 20% by weight water, 14.3% by weight tobacco and 0.7% by weight agar.

Advantageously, the cartridge does not comprise a transport element or mechanism for transporting the aerosol-former to a heat source or heater. The gel is advantageously heated in situ to generate a desired aerosol. In this context in situ means in the same position within the cartridge. There is no requirement for a capillary wick or pump. Advantageously, the system does not comprise an additional non-volatile structure within the substrate cartridge for holding or retaining a liquid or gel in proximity to the heater.

The device may comprise a device housing having a cavity for receiving the cartridge. The cavity of the device may be substantially cylindrical. Preferably, the cavity has a diameter substantially equal to or slightly greater than the diameter of the cartridge.

The device may comprise a device body holding the power supply and the heater. The aerosol-generating device may further comprise a mouthpiece separate to the device body. The mouthpiece may be configured for engagement with the device body. The device body may be configured to receive the cartridge in a cavity of the device body. By providing a reusable mouthpiece, separate to the consumable portion, the construction of the consumable portion can be simple.

Advantageously, at least one wall of the substrate cartridge is in thermal contact with the heater. The at least one wall of the substrate cartridge may be positioned between the heater and the aerosol-forming substrate. Preferably, the at least one wall of the substrate cartridge is in direct contact with the heater. The gel within the substrate cartridge can then be heated by conduction through the external wall. Advantageously, the substrate cartridge comprises at least one liquid impermeable and vapour impermeable external wall defining a blind cavity, wherein the aerosol-forming substrate is held in the device body.

The cartridge may have any suitable shape.

Preferably, the cartridge is substantially cylindrical. As used herein with reference to the present invention, the terms "cylinder" and "cylindrical" refer to a substantially right circular cylinder with a pair of opposed substantially planar end faces.

The cartridge may have any suitable size.

The cartridge may have a length of, for example, between about 5 mm and about 30 mm. In certain embodiments the cartridge may have a length of about 12 mm.

The cartridge may have a diameter of, for example, between about 4 mm and about 10 mm. In certain embodiments the cartridge may have a diameter of about 7 mm.

The substrate cartridge or cartridge may comprise a housing. The housing of the cartridge may be formed from one or more materials. Suitable materials include, but are not limited to: metal, aluminium, polymer, polyether ether ketone (PEEK), polyimides, such as Kapton^{®}, polyethylene terephthalate (PET), polyethylene (PE), polypropylene (PP), polystyrene (PS), fluorinated ethylene propylene (FEP), polytetrafluoroethylene (PTFE), epoxy resins, polyurethane resins and vinyl resins.

The housing of the cartridge may be formed from one or more thermally conductive materials. The interior of the cartridge may be coated or treated to comprise one or more thermally conductive materials. Use of one or more thermally conductive materials to form the cartridge or coat the interior of the cartridge can advantageously increase heat transfer from the heater to the gel. Suitable thermally conductive materials include, but are not limited to, metals such as, for example, aluminium, chromium, copper, gold, iron, nickel and silver, alloys, such as brass and steel and ceramics, or combinations thereof. Advantageously, at least one wall of the housing has a thermal conductivity greater than 10 Watts per metre per Kelvin at room temperature. In a preferred embodiment, the housing comprises a least one wall formed from aluminium.

In embodiments in which the cartridge is configured to be heated inductively, the housing of the cartridge may comprise a susceptor, for example a susceptor layer. The susceptor layer may for example form a wall of the housing or may be a coating applied to the interior or exterior of the housing. A susceptor may be located within a chamber in the cartridge. For example, the gel may comprise a susceptor material.

Cartridges for use in aerosol-generating systems according to the present invention may be formed by any suitable method. Suitable methods include, but are not limited to, deep drawing, injection moulding, blistering, blow forming and extrusion.

The cartridge may comprise a mouthpiece configured to allow a user to puff on the mouthpiece to draw aerosol into their mouth or lungs. Where the cartridge comprises a mouthpiece, the mouthpiece may comprise a filter. The filter may have a low particulate filtration efficiency or very low particulate filtration efficiency. Alternatively, the mouthpiece may comprise a hollow tube. The mouthpiece may comprise an airflow modifier, for example a restrictor.

The cartridge may be provided within a mouthpiece tube. The mouthpiece tube may comprise an aerosol-forming chamber. The mouthpiece tube may comprise an airflow restrictor. The mouthpiece tube may comprise a filter. The mouthpiece tube may comprise a cardboard housing. The mouthpiece tube may comprise one or more vapour impermeable elements within the cardboard tube. The mouthpiece tube may have a diameter similar to a conventional cigarette, for example around 7mm. The mouthpiece tube may have a mouth end configured to be placed in a user's mouth for inhalation of aerosol therethrough. The cartridge may be held in the mouthpiece tube, for example at an opposite end to the mouth end.

An open end of the substrate cartridge may be sealed by one or more frangible sealing elements.

The one or more frangible barriers may be formed from any suitable material. For example, the one or more frangible barriers may be formed from a foil or film, for example comprising a metal. Where the cartridge comprises one or more frangible barriers sealing one or both of the first chamber and the second chamber, the device body preferably further comprises a piercing member configured to rupture the one or more frangible barriers.

Alternatively or in addition, the substrate container may be sealed by one or more removable barriers. For example, the substrate container may be sealed by one or more peel-off seals.

The one or more removable barriers may be formed from any suitable material. For example, the one or more removable barriers may be formed from a foil or film. for example comprising a metal.

An open end of the substrate container may be sealed by a vapour permeable element, for example a membrane or mesh configured to allow the escape of vapour from the substrate container through the membrane or mesh. Alternatively, the substrate container may be sealed by a pressure activated valve that allows for the release of vapour through the valve when a pressure difference across the valve exceeds a threshold pressure difference.

The substrate container may comprise a first chamber, containing the gel and a second chamber separate to the first chamber. The second chamber may contain the same gel as the first chamber or may contain a different gel or different material to the first chamber.

The first and second chambers may be fixed together permanently or they may be separable from one another. The first and second chambers may be provided separately and fixed together by a user using a suitable mechanical interlock, such as a snap fitting or a screw fitting. Alternatively, the first and second chambers may remain separate during use.

By providing the first and second chambers separately, a "mix and match" type set of choices may be made available to a user. The contents of the first chamber may provide a particular dosage of a target compound for delivery to a user, such as nicotine, and may provide a particular density of aerosol, and a range of options may be made available to the user. The contents of the second chamber may primarily provide flavour compounds, and a range of options for the second chamber may be available to the user. The user can choose one chamber from the range of first chambers and one chamber from the range of second chambers and may fit them together to form a complete cartridge.

Even when the first and second chambers are provided together and permanently fixed to one another, the same mix and match approach may be taken by a manufacturer to provide a range of different cartridges.

The first and second chambers may be of the same size and shape as one another or they may have a different size or shape to one another. The size and shape of the first and second chamber may be chosen to suit their contents, and to provide for a particular heating rate in use.

It is also possible to have more than two chambers. It may be desirable to have three or more chambers in the cartridge, with at least two of the chamber having different contents.

The first and second chambers may advantageously contain different compositions. Both the first and second chambers may contain a gel. Advantageously, neither the first chamber nor the second chamber contains a liquid at room temperature. Advantageously, neither the first chamber nor the second chamber comprises a liquid retention material or a wicking material.

The first and second chambers may be positioned side by side or one within the other or may be arranged in series such that an air flow can pass first through one chamber and then through the other.

The cartridge may comprise a slot between the first and second chambers. The slot may be configured to receive a heating element. The heating element may be received in the slot for example when the cartridge is installed in an aerosol-forming device. The provision of a slot into which a heating element is received may provide for efficient heating by facilitating that heat energy from the heating element is passed directly to the interior of the substrate container rather than for example heating other elements of the system or the ambient air. Advantageously the slot is a blind slot. Blind in this context means closed at one end. The provision of a blind slot allows the heating element to be shielded from the vapour or aerosol generated by the system and can help to prevent the build-up of condensates on the heater.

Where the substrate comprises first and second chambers, the slot may be provided between the first the second chambers. For example, the slot may be provided within a wall separating the first and second chambers.

The electrical heater may comprise a resistive heater. The electrical heater may comprise one or more heating elements.

The electric heating element may comprise one or more external heating elements, one or more internal heating elements, or one or more external heating elements and one or more internal heating elements. In this context, external means outside of the cavity and internal means inside of the cavity of the device body.

The one or more external heating elements may comprise an array of external heating elements arranged around the inner surface of the cavity. In certain examples, the external heating elements extend along the longitudinal direction of the cavity. With this arrangement, the heating elements may extend along the same direction in which the cartridge is inserted into and removed from the cavity. This may reduce interference between the heating elements and the cartridge. In some embodiments, the external heating elements extend along the length direction of the cavity and are spaced apart in the circumferential direction. Where the heating element comprises one or more internal heating elements, the one or more internal heating elements may comprise any suitable number of heating elements. For example, the heating element may comprise a single internal heating element. The single internal heating element may extend along the longitudinal direction of the cavity.

The electric heating element may comprise an electrically resistive material. Suitable electrically resistive materials include but are not limited to: semiconductors such as doped ceramics, electrically "conductive" ceramics (such as, for example, molybdenum disilicide), carbon, graphite, metals, metal alloys and composite materials made of a ceramic material and a metallic material. Such composite materials may comprise doped or undoped ceramics. Examples of suitable doped ceramics include doped silicon carbides. Examples of suitable metals include titanium, zirconium, tantalum and metals from the platinum group. Examples of suitable metal alloys include stainless steel, Constantan, nickel-, cobalt-, chromium-, aluminium- titanium- zirconium-, hafnium-, niobium-, molybdenum-, tantalum-, tungsten-, tin-, gallium-, manganese- and iron-containing alloys, and super-alloys based on nickel, iron, cobalt, stainless steel, Timetal^{®}, iron-aluminium based alloys and iron-manganese-aluminium based alloys. Timetal^{®} is a registered trade mark of Titanium Metals Corporation, 1999 Broadway Suite 4300, Denver Colorado. In composite materials, the electrically resistive material may optionally be embedded in, encapsulated or coated with an insulating material or vice-versa, depending on the kinetics of energy transfer and the external physicochemical properties required. The heating element may comprise a metallic etched foil insulated between two layers of an inert material. In that case, the inert material may comprise Kapton^{®}, all-polyimide or mica foil. Kapton^{®} is a registered trade mark of E.I. du Pont de Nemours and Company, 1007 Market Street, Wilmington, Delaware 19898, United States of America. A flexible heating element of this type may be conformed to the shape of the cavity and may extend around the periphery of the cavity.

The electric heating element may be formed using a metal having a defined relationship between temperature and resistivity. In such embodiments, the metal may be formed as a track between two layers of suitable insulating materials. An electric heating element formed in this manner may be used both as a heater and a temperature sensor.

Where the electric heating element comprises a susceptor, the aerosol-generating device body preferably comprises an inductor arranged to generate a fluctuating electromagnetic field within the cavity and an electrical power supply connected to the inductor. The inductor may comprise one or more coils that generate a fluctuating electromagnetic field. The coil or coils may surround the cavity.

Preferably, the device body is capable of generating a fluctuating electromagnetic field of between 1 and 30 MHz, for example, between 2 and 10 MHz, for example between 5 and 7 MHz. Preferably the device body is capable of generating a fluctuating electromagnetic field having a field strength (H-field) of between 1 and 5 kA/m, for example between 2 and 3 kA/m, for example about 2.5 kA/m.

The aerosol-generating system according to the present invention may comprise a single heater. This advantageously provides for a simple device construction. The single heater may be configured as an external heater that in use is positioned externally to the cavity. Alternatively, the single heater may be configured as an internal heater that in use is positioned internally to the cavity and received in a slot in the cartridge. Preferably, the single heater is configured as an internal heater.

Where the single heater is configured as an internal heater, the aerosol-generating device may advantageously comprise guide means to facilitate proper alignment of the internal heater with the cartridge.

Preferably, the single heater is an electric heating element comprising an electrically resistive material. The electric heating element may comprise a non-elastic material, for example a ceramic sintered material, such as glass, alumina (Al₂O₃) and silicon nitride (Si₃N₄), or printed circuit board or silicon rubber. Alternatively, the electric heating element may comprise an elastic, metallic material, for example an iron alloy or a nickel-chromium alloy.

The single heater may have any shape suitable to heat the cartridge. The electrical heater may be positioned between first and second chambers of the cartridge when the cartridge is connected to or received in the device body. Preferably, the heater does not project from the aerosol-generating device.

The electrical heater may surround the substrate cartridge. The electrical heater may comprise one or more electrically resistive tracks on a flexible substrate. Preferably, the electrical heater comprises one or more electrically resistive tracks on a rigid substrate material. Preferably, the electrical heater projects into the cavity of the device.

The aerosol-generating system of the present invention may further comprise one or more temperature sensors configured to sense the temperature of at least one of the electrical heater elements. In such embodiments, the system may comprise a controller and the controller may be configured to control a supply of power to the electrical heater based on the sensed temperature. Advantageously, the controller is configured to supply power to the heater continuously after activation of the system rather than in response to detected user puffs.

The system may comprise electronic circuitry to control the supply of power to the electrical heater. The electronic circuitry may be a simple switch. Alternatively the electronic circuitry may comprise one or more microprocessors or microcontrollers. The electronic circuitry may be programmable.

The electrical power supply may be a DC voltage source. In preferred embodiments, the power supply is a battery. For example, the power supply may be a Nickel-metal hydride battery, a Nickel cadmium battery, or a Lithium based battery, for example a Lithium-Cobalt, a Lithium-Iron-Phosphate or a Lithium-Polymer battery. The power supply may alternatively be another form of charge storage device such as a capacitor. The power supply may require recharging and may have a capacity that allows for the storage of enough energy for use of the aerosol-generating device with one or more aerosol-generating articles.

Preferably, the aerosol-generating system is configured to generate an aerosol for inhalation by a user. The aerosol-generating system may be a handheld system and may comprise a mouthpiece on which a user sucks or draws in use.

Advantageously, the system does not comprise a transport mechanism for transporting the aerosol-former to the heater. The contents of the cartridge are advantageously heated *in situ* to generate a desired aerosol. In this context *in situ* means in the same position within the first and second chambers that the contents are held prior to use. There is no requirement for a capillary wick or pump.

Preferably, the aerosol-generating device is a portable or handheld aerosol-generating device that is comfortable for a user to hold between the fingers of a single hand.

The aerosol-generating device may be substantially cylindrical in shape. The aerosol-generating device may have a length of between approximately 70 millimetres and approximately 120 millimetres.

In another aspect of the invention, there is provided a cartridge for an aerosol-generating system, the aerosol-generating system comprising a heater, the cartridge comprising:
a substrate cartridge containing an aerosol-forming substrate in the form of a thermoreversible gel that is solid at room temperature, wherein the cartridge is configured to removably connect to or be received in a body of the aerosol-generating system and wherein the cartridge comprises a slot configured to receive the heater.

Features of the substrate cartridge and cartridge described in relation to the first aspect of the invention may apply to the cartridge of the second aspect of the invention. In particular, the substrate cartridge may comprise at least one liquid and vapour impermeable external wall defining a blind cavity, wherein the aerosol-forming substrate is contained in the blind cavity. "Blind" in this context means closed at on end. The cartridge may comprise a mouthpiece tube, wherein the substrate cartridge is held in the mouthpiece tube. The mouthpiece tube may have a mouth end for insertion into a user's mouth. The mouthpiece tube may comprise an air flow modifier, such as a restrictor.

The invention will now be further described with reference to the accompanying drawings which further illustrate embodiments according to the present invention and in which:
Figure 1 is a schematic illustration of an aerosol-generating system in accordance with a first embodiment of the invention;
Figure 2a is a perspective view of a mouthpiece portion in accordance with a first embodiment of the invention;
Figure 2b is a bottom perspective view of a cartridge housing in accordance with a first embodiment of the invention;
Figure 2c is a top perspective view of the cartridge of Figure 2b;
Figure 2d is a cross section view of the cartridge of Figure 2b;
Figure 3 illustrates an embodiment in which a mouthpiece portion pierces a frangible seal on a cartridge in accordance with the invention;
Figure 4 is a schematic illustration of an aerosol-generating system in accordance with a further embodiment of the invention;
Figure 5a is a schematic illustration of a cartridge held within a mouthpiece tube in accordance with a further embodiment of the invention;
Figure 5b is an exploded view of the elements within the mouthpiece tube of Figure 5a;
Figure 6 is an illustration of the airflow through the mouthpiece tube of Figure 6a;
Figure 7a is a schematic illustration of an aerosol-generating device in accordance with a further embodiment of the invention;
Figure 7b shown the device of Figure 7a with a cartridge received in a cavity of the device; and
Figure 8 shows the cartridge of Figure 7b in detail.

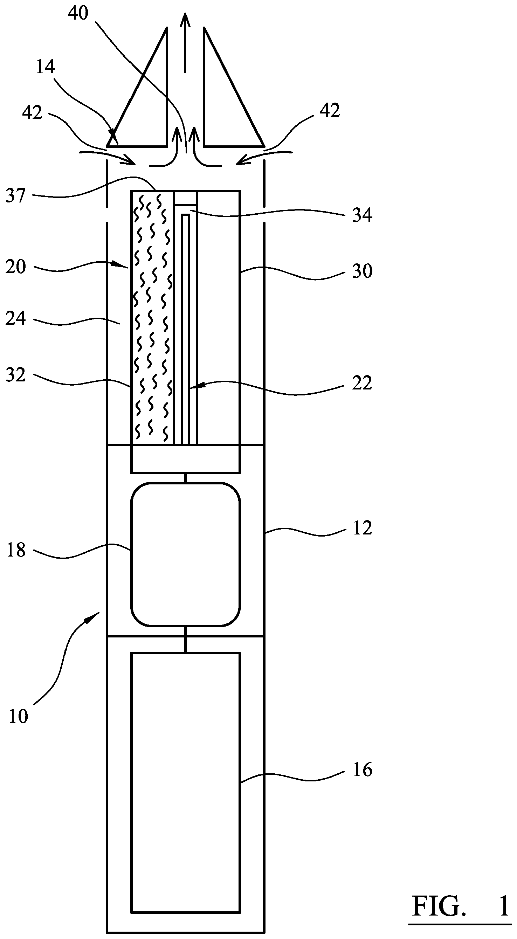

Figure 1 is a schematic illustration of an aerosol-generating system in accordance with a first embodiment of the invention. The system comprises an aerosol-generating device 10 and a replaceable cartridge 20. The aerosol-generating device comprises a device body 12 and a mouthpiece portion 14.

The device body 12 comprises a power supply, which is a lithium ion battery 16 and electronic control circuitry 18. The device body also includes heater 22, which is in the form a blade that projects into a cavity 24 in the housing of the device body. The heater is an electric heater comprising an electrically resistive track on a ceramic substrate material. The control circuitry is configured to control the supply of power from the battery 16 to the electric heater 22.

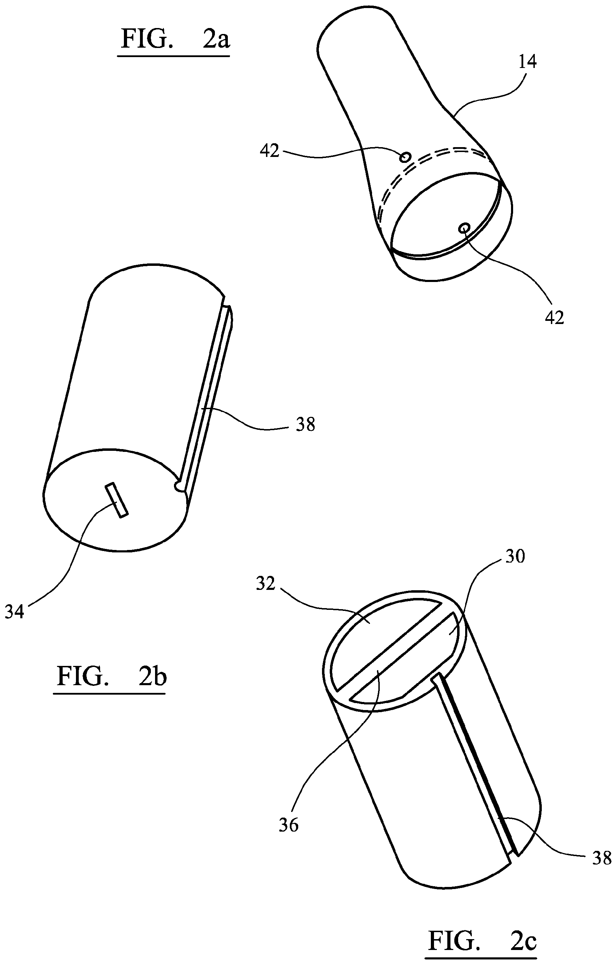

The mouthpiece portion 14 engages the device body using a simple push fitting, although any type of connection, such as a snap fitting or screw fitting may be used. The mouthpiece portion in this embodiment is simply a tapered hollow tube, without any filter elements, and is shown in more detail in Figure 2a. However, it is possible to include one or more filter elements in the mouthpiece portion. The mouthpiece portion comprises air inlet holes 42 and encloses an aerosol-forming chamber 40 (shown in Figure 1) in which vapour can condense in an airflow prior to entering a user's mouth.

The cartridge 20 comprises a housing defining two blind chambers. The two chambers 30, 32 are open at a mouthpiece end. A membrane 37 (shown in Figure 1) seals the open end of the chambers. A removable seal may be provided over the membrane that a user peels off before use. A blind slot 34 is provided between the two chambers for the heater 22 to be received in. The blind slot 34 is closed at the mouthpiece end. A first chamber 30 holds a first gel, containing nicotine and aerosol-former, and the second chamber 32 holds a second gel, containing shredded tobacco leaves.

Figure 2b is a bottom perspective view of the cartridge housing. Figure 2c is a perspective view of the cartridge housing. The cartridge 20 has a generally cylindrical shape. The first and second chambers are of equal size and shape and are separated by a dividing wall 36. The blind slot 34 is within the dividing wall 36. A channel 38 is provided in a wall of the cartridge housing to engage a corresponding rib in the cavity 24. This ensures that the cartridge can only be inserted into the cavity 24 in one orientation, in which the heater blade is received in the slot 34.

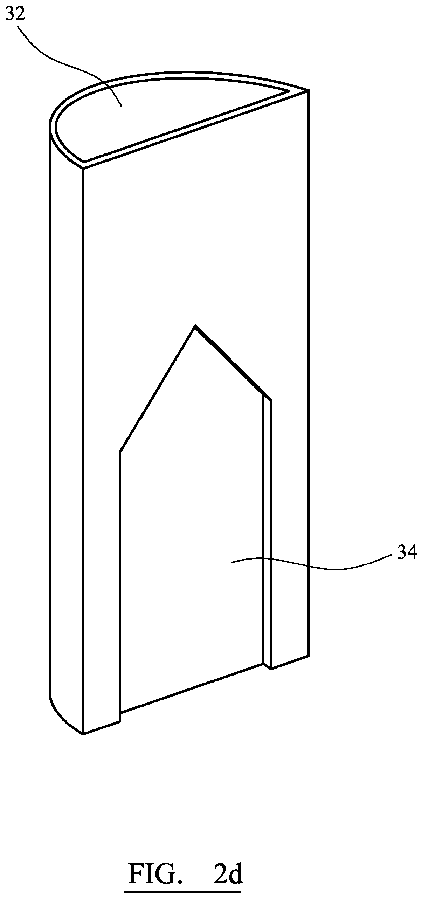

Figure 2d is a cross section through the cartridge housing of Figures 2b and 2c showing the shape of the blind slot 34. The shape of the slot matches the blade shape of the heater.

The first gel in the first chamber 30 comprises one or two aerosol formers such as glycerin and polyethylene glycol. The relative concentration of the aerosol formers can be adapted to the particular requirements of the system. In this embodiment the gel in the first chamber 30 comprises (by weight): 2% nicotine, 70% glycerin, 27% water, 1% agar.

The gelling agent is preferably agar. It has the property of melting at temperatures above 85°C and turning back to gel at around 40°C. This property makes it suitable for hot environments. The gel will not melt at 50°C, which is useful if the system is left in a hot automobile in the sun, for example. A phase transition to liquid at around 85°C means that the gel only needs to be heated to a relatively low-temperature to induce aerosolization, allowing low energy consumption. It may be beneficial to use only agarose, which is one of the components of agar, instead of agar.

The second gel in the second chamber 32 comprises (by weight): 65% glycerin, 20% water, 14.3% solid powdered tobacco, 0.7% agar.

Further or different flavors, such as menthol, can be added either in water or in propylene glycol or glycerin prior to the formation of the either of the gels.

The amount of gel provided in each cartridge can also be chosen to suit particular needs. Each cartridge may contain enough gel to provide a single dose or usage session for a user or may contain sufficient gel for several or many doses or usage sessions.

In operation, the system is configured to operate in a continuous heating mode. This means that the heater 22 heats the cartridge throughout an operating session rather than in response to sensed user puffs. The user turns the system on using a simple switch (not shown) and the heater heats the cartridge. A temperature sensor may be included in the system so that a user can be provided with an indication of when an operating temperature has been reached, at which aerosol is generated. The gels become liquid upon heating above 85°C. Aerosol containing nicotine and glycerin is generated at temperatures between 180°C to 250°C. During operation, the heater operates at approximately 250°C.The heater may operate for a fixed time period after activation, say 6 minutes, or may operate until a user switches the system off. The operating time may depend on the amount of gel contained within the cartridge.

The cartridge housing is formed of aluminium, which is a good thermal conductor. The heater is never in contact with the gel or any generated vapour or aerosol. It is held in the blind slot 34 and so is isolated from the generated aerosol. This ensures that there is no build-up of condensates on the heater, which might lead to the generation of undesirable compounds in operation.

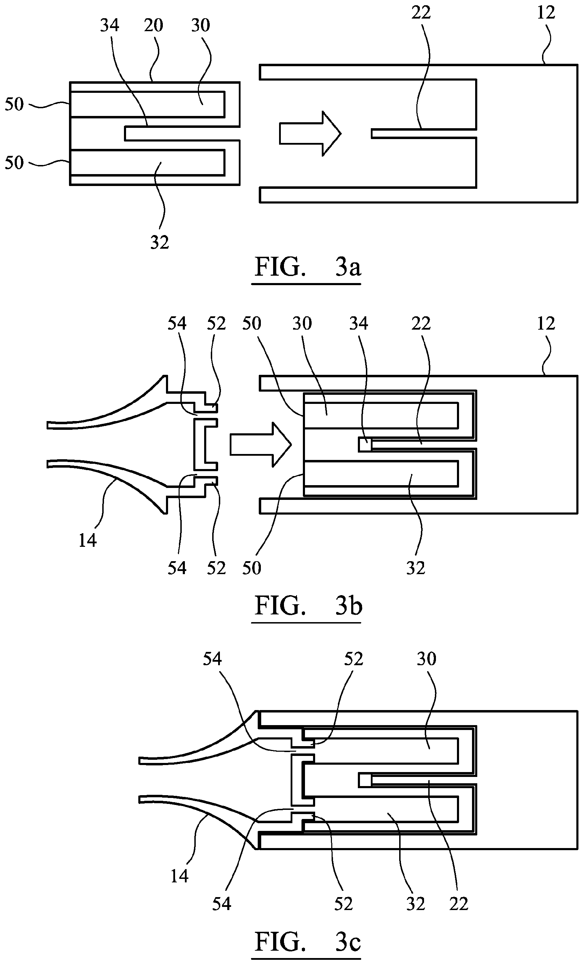

Figure 3 illustrates an embodiment in which the chambers of the cartridge are sealed by a frangible sealing element. The mouthpiece portion is used to pierce the sealing element to allow vapour generated in the chambers to escape from the two chambers.

Figure 3a illustrates the insertion of the cartridge 20 into the device 12. As in Figure 1, the cartridge comprises first and second chambers 30, 32 and a blind slot 34 between the chambers. The chambers are sealed by sealing element 50.

Figure 3b shows the cartridge inserted into the device, with the heater 22 received in the slot 34 between the chambers. A mouthpiece portion 14 is then connected to the device body portion 12. Figure 3b illustrates the direction of insertion of the mouthpiece portion. The mouthpiece portion is provided with piercing elements 52 which act to pierce the frangible sealing element and provide an escape passage 54 for vapour generated in the first and second chambers.

Figure 3c shows the mouthpiece portion 14 in a fully inserted position, with the piercing elements 52 extending into the first and second chambers and allowing vapour to escape from the first and second chambers 30, 32, into an aerosol-forming chamber in the mouthpiece portion. The vapour cools and is entrained in an airflow in the mouthpiece portion to form an aerosol, before being inhaled by the user. As in the embodiment of Figure 1, the mouthpiece portion may be provided with air inlets. Alternatively or in addition, an airflow path into the mouthpiece portion may be provided through the device. Alternatively or in addition, an airflow path may be provided through the first and second chambers.

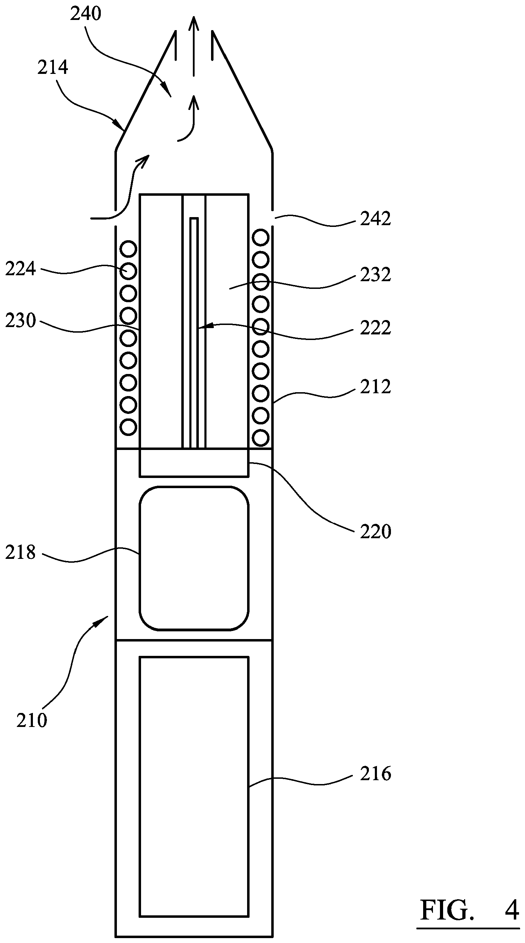

Figure 4 is a schematic illustration of an aerosol-generating system in accordance with a still further embodiment of the invention. The embodiment of Figure 4 operates by using induction heating rather than by using resistive heating. Instead of using a resistive heater either around or inside the cavity in which the cartridge is received, the device body comprises an inductor coil surrounding the cavity and a susceptor is provided in the cavity, in this example as part of the cartridge.

The device body 212 comprises a power supply, which is a lithium ion battery 216 and electronic control circuitry 218. The device body also includes an induction coil 224, which extends around a cavity in the housing of the device body. The device body also comprises electronic circuitry 220 to generate an AC signal which is provided to the induction coil 224.

The mouthpiece portion 214 is similar to the mouthpiece portion shown in Figure 1 and encloses an aerosol-forming chamber 240. In this example air inlets 242 are provided at the junction of the mouthpiece portion and the device body.

The cartridge of Figure 4 is similar to the cartridge shown in Figure 1.The composition of the gels in the two chambers of the cartridge may be the same as in the embodiment of Figure 1. However, rather than having a blind cavity for receiving a heater, the wall of the cartridge separating the two chambers comprises a susceptor material 222, such as a layer of iron, that heats up in the alternating magnetic field. The susceptor material in this example is provided as part of the cartridge rather than part of the device body, but it is possible for the susceptor material to be provided as part of the device body or both in the cartridge and the device body. The entire cartridge may be formed from a susceptor material, or a susceptor material may be provided as a coating on one of more surfaces of the cartridge. It is also possible to provide susceptor material within the first and second chambers, suspended in the gel or other material contained there.

A sealing element is provided to seal the first and second chambers in the same manner as described with reference to Figure 1. A cartridge piercing arrangement similar to that shown in Figure 3 may be used to open the cartridge using the mouthpiece portion 114, with suitable adaptations made for the different airflow path. Alternatively, a simple peelable seal may be used and a vapour permeable membrane provided across the open end of the first and second chambers 230, 232.

In operation, the system is configured to operate in a continuous heating mode as in the embodiment of Figure 1. This means that when a user switches the device on, the device supplies an AC signal to the induction coil in order to generate an alternating magnetic field in the cavity. This induces current flow in the susceptor resulting in a heating of the susceptor. If a ferromagnetic material is used as the susceptor, hysteresis losses may also contribute to the heating. The induction coil may be described as an induction heater in this context. By controlling the magnitude and frequency of the AC signal, the temperature within the first and second chambers can be controlled. A temperature sensor may be provided within the cavity and a feedback control loop used. Again the induction heater may operate for a fixed time period after activation, say 6 minutes, or may operate until a user switches the system off.

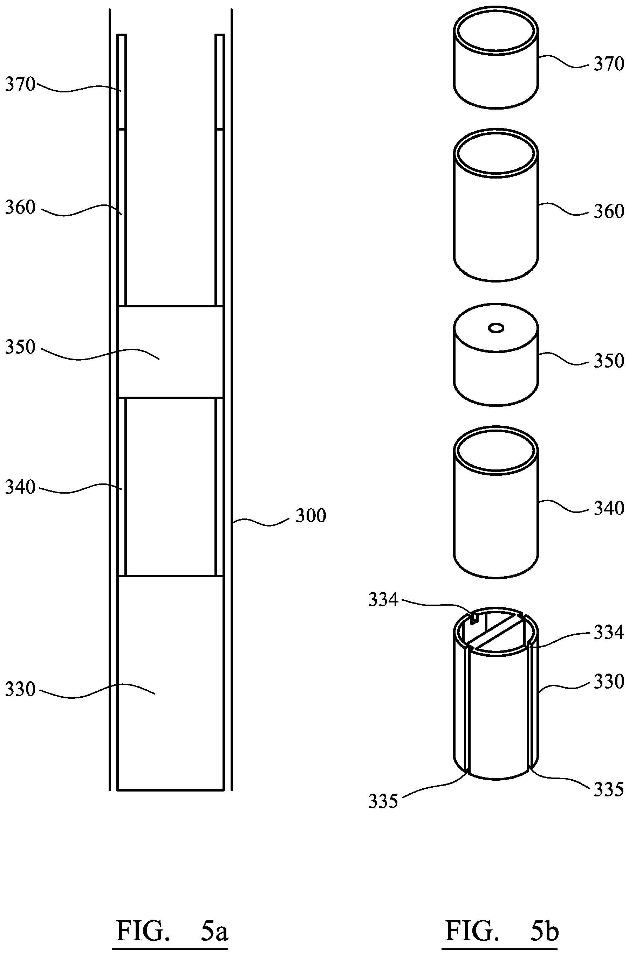

Figure 5a is a schematic illustration of a further embodiment of the invention. In the embodiment of Figure 5a, the cartridge 330 is held within a mouthpiece tube 300. A flow restrictor 350 and lining tubes 340, 360, 370 are also held within the mouthpiece tube. The components held within the mouthpiece tube 330 are shown in an exploded view in Figure 5b.

The cartridge 330 is similar to the cartridge shown in Figure 2c. However, the cartridge 330 has no membrane or sealing element but includes airflow channels 335 formed in the walls of the cartridge and air inlets 334 at the top of the airflow channels to allow air into the open ends of the first and second chambers.

The mouthpiece tube is formed from cardboard and has a diameter of 6.6mm and a length of 45 mm. Lining tubes 340 are formed from polyetheretherketone (PEEK) and are provided to prevent the cardboard mouthpiece tube from absorbing moisture from within the mouthpiece tube. The lining tubes can be made very thin, in this embodiment having a thickness of 0.3mm. A restrictor 350 is provided to restrict the airflow to ensure mixing of air with vapour from the cartridge and ensure the generation of an aerosol within the space following the restrictor, in lining tube 360.

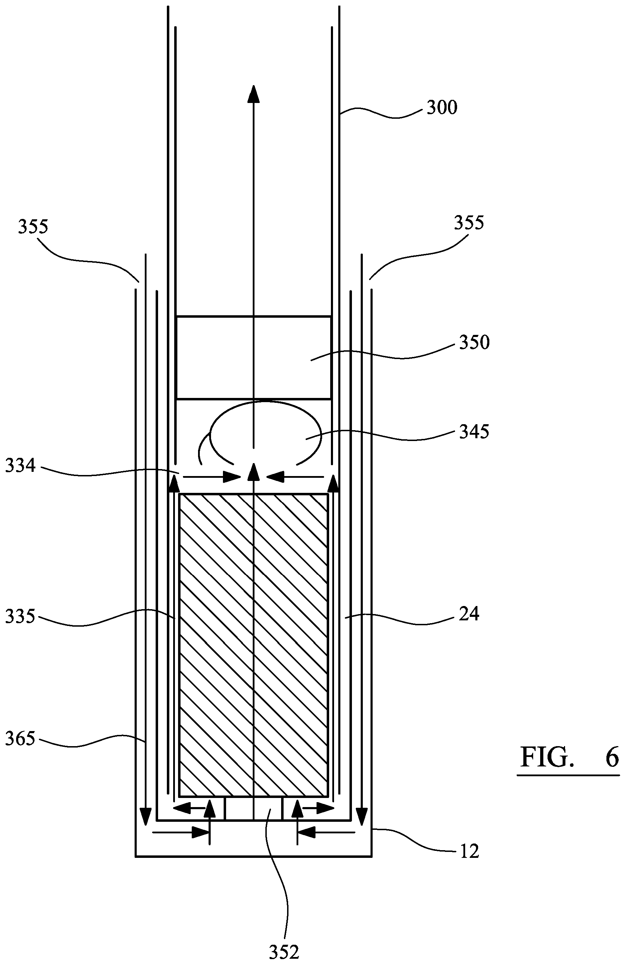

Figure 6 illustrates the airflow within the mouthpiece tube of Figure 5a during operation. The mouthpiece tube is shown within the cavity 24 of a device 12 of the type shown in Figure 1. But the device 12 of Figure 6 does not have a mouthpiece 14. Figure 6 illustrates only the end of the device that receives the mouthpiece tube. The battery and control circuitry is not shown. The device includes device air inlets 355 that allow air into an internal air flow passage 365 formed in the device around the periphery of the cavity 24. A spacer element 352 is positioned in a base of the cavity to allow air to flow from the internal airflow passage 365 into the cavity 24 and then into the airflow channels 335 in the cartridge 330 and through the air inlets 334 into the interior of the mouthpiece tube.

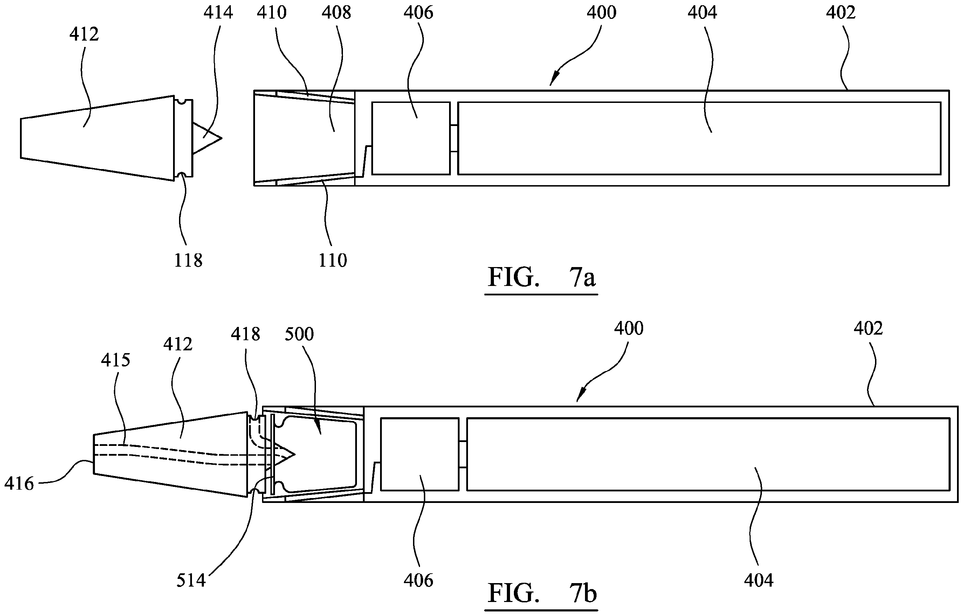

The cartridge shown in Figures 5a and 5b may be heated by heater of the type shown in Figure 1 or of the type shown in Figures 4 or 7a (described below). In operation, the system is configured to operate in a continuous heating mode as in Figure 1. This means that the heater heats the cartridge throughout an operating session rather than in response to sensed user puffs. The user turns the system on using a simple switch (not shown) and the heater heats the cartridge. The gels in the first and second chambers become liquid upon heating and vapour containing nicotine and glycerin is generated at temperatures between 180°C to 250°C.

When the system is at the operating temperature, the user sucks on a mouth end of the mouthpiece tube to draw air through the mouthpiece tube. Air is drawn into a distal end of the mouthpiece tube, opposite the mouthpiece end from the internal passage 365. The air travels up the airflow channels 335 and through air inlets 334 into space 345. The air mixes in space 345 with vapour from the first and second chambers. The mixed air and vapour then passes through the restrictor 350, after which it cools to form an aerosol before being drawn into a user's mouth. After operation, the mouthpiece tube, including the cartridge, can be withdrawn from the device and disposed of. Mouthpiece tubes of this type may be sold in packs to provide for multiple operations of the system.

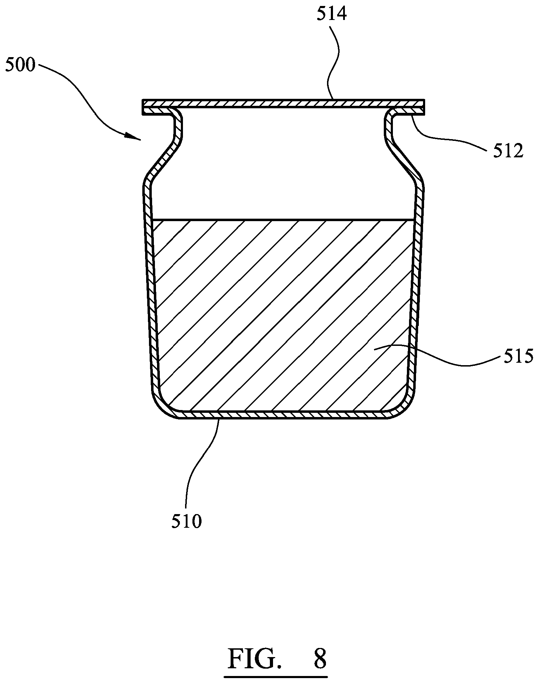

Figure 7a is a schematic illustration of an aerosol-generating device in accordance with a further embodiment of the invention. Figure 7a shows a cross-sectional view of an aerosol-generating device 400 for use with a container or cartridge 500 as shown in Figure 8. The aerosol-generating device comprises an outer housing 402, containing a power supply 404 such as a rechargeable battery and control circuitry 406. The housing 402 further comprises a cavity 408 configured to receive a container 500. A heater 410 extends around the periphery of the cavity 108. The control circuitry is connected to the heater 410. The heater is formed from one or more metal heating tracks sandwiched between two layers of flexible, thermal stable substrate material, such as polyimide. The aerosol-generating device 400 further comprises a mouthpiece 412 attachable to a proximal end of the aerosol-generating device housing 402 by a push fitting or screw fitting. The mouthpiece comprises a piercing portion 414, air inlets 418 and an air outlet 416.

The container or cartridge 500 that the user places in the cavity 408 of the device, is shown in Figure 8. The container has a housing 510 formed from aluminium, which is a good thermal conductor. The housing of the container is in the form of a cup, that defines a blind cavity. The housing 510 may be manufactured using suitable known techniques, such as deep drawing. The container contains a gel 515. In this embodiment the gel comprises 2% by weight nicotine, 70% by weight glycerol, 27% by weight water and 1% by weight agar. In another embodiment, the gel comprises 65% by weight glycerol, 20% by weight water, 14.3% by weight tobacco and 0.7% by weight agar. The gel is sealed in the cavity of the container by a frangible sealing foil 514. The sealing foil is welded, heat sealed or adhered to a lip 512 of the housing 510. This type of container can be made very inexpensively.

Figure 7b shows a cross-sectional view of the aerosol-generating device 400 with a container 500 received in the cavity 408 of the housing. In use, a user inserts the container 500 into the cavity 108 of the aerosol-generating device 400, and then attaches the mouthpiece 412 to the housing 402. By attaching the mouthpiece, the piercing portion 414 pierces the sealing foil 514 of the container, and forms an airflow pathway 415 from the air inlets 418, through the container to the air outlet. The user then presses a button (not shown) to activate the device. After activating the device, the heater is supplied with power by the control electronics 406 from the power supply 404. The heater then directly heats the external wall of the cartridge. When the temperature of the container 500 reaches the operating temperature of about 250 degrees Celsius, the user is informed by means of an indicator (not shown) that the user may then draw on the mouthpiece at outlet 416. When the user draws on the mouthpiece, air enters the air inlets 418, proceeds through the mouthpiece and into the container 500, entrains vapourised gel, and then exits to the user's mouth through the air outlet 416 in the mouthpiece. The heater may operate for a fixed time period after activation, say 6 minutes, or may operate until a user switches the system off.

When the gel in the cartridge has become exhausted, the cartridge can be removed by the user and replaced by a new cartridge.

The embodiments described have each been described as configured to operate a continuous heating scheme, in which the heater is activated for a predetermined time period during which a user may take several puffs. However, the systems described may be configured to operate in different ways. For example, power may be provided to the heater or induction coil for only the duration of each user puff, based on signals from an airflow sensor within the system. Alternatively, or in addition, power to the heater or induction coil may be switched on and off in response to user actuation of a button or switch.

The figures show particular embodiments of the invention. But it should be clear that changes may be made to the described embodiments within the invention as defined in the claims. In particular, different arrangements for airflow through the system may be provided and different heating arrangements can be envisaged, such as non-electrical heaters.

## Claims

1. An aerosol-generating system comprising:
a device (10) comprising a power supply (16) and an electrical heater, the electrical heater (22) connected to the power supply (16); and
a substrate cartridge (20) containing an aerosol-forming substrate in the form of a thermoreversible gel that is solid at room temperature;
wherein the substrate cartridge (20) is configured to be inserted into or connected to the device prior to use and removed or disconnected from the device (10) after use.

2. An aerosol-generating system according to claim 1, wherein the electrical heater (22) does not contact the aerosol-forming substrate.

3. An aerosol-generating system according to claim 1 or 2, wherein at least one wall of the substrate cartridge (20) is provided between the electrical heater (22) and the aerosol-forming substrate.

4. An aerosol-generating system according to any preceding claim, wherein the electrical heater (22) comprises a resistive heating track in or on a rigid substrate material.

5. An aerosol-generating system according to any preceding claim, wherein the electrical heater (22) is received in a slot (34) in the cartridge.

6. An aerosol-generating system according to claim 5, wherein the slot (34) is a blind slot.

7. An aerosol-generating system according to any preceding claim, wherein at least one wall of the cartridge (20) is in thermal contact with the heater (22).

8. An aerosol-generating system according to any preceding claim, wherein the substrate cartridge (20) comprises at least one liquid and vapour impermeable external wall defining a blind cavity (34), wherein the aerosol-forming substrate is contained in the blind cavity (34).

9. An aerosol-generating system according to claim 8, wherein the blind cavity (34) is sealed by a frangible, removable or vapour-permeable sealing element (50).

10. An aerosol-generating system according to any preceding claim, wherein the device (10) comprises a mouthpiece portion (14) separate to the cartridge (20).

11. An aerosol-generating system according to any preceding claim, wherein the substrate cartridge (20) comprises a first chamber (30) and a second chamber (32) separate to the first chamber (30).

12. An aerosol-generating system according to claim 11, wherein at least a portion of the electrical heater (22) is positioned between the first (30) and second (32) chambers.

13. An aerosol-generating system according to any preceding claim, wherein the gel comprises a source of nicotine or a tobacco product.

14. A cartridge for an aerosol-generating system, the aerosol-generating system comprising a heater (22), the cartridge comprising:
a substrate cartridge (330) containing an aerosol-forming substrate in the form of a thermoreversible gel that is solid at room temperature, wherein the cartridge is configured to removably connect to or be received in a body of the aerosol-generating system and wherein the cartridge comprises a slot (34) configured to receive the heater (22).

15. A cartridge according to claim 14, wherein the substrate cartridge comprises at least one liquid impermeable and vapour impermeable external wall defining a blind cavity (30,32), wherein the aerosol-forming substrate is contained in the blind cavity (30,32).

16. A cartridge according to claim 14 or 15, wherein the cartridge comprises a mouthpiece tube (300), wherein the substrate cartridge (330) is held in the mouthpiece tube (300), and wherein the mouthpiece tube (300) has a mouth end for insertion into a user's mouth.

17. A cartridge according to claim 16, wherein the mouthpiece tube (300) comprises an air flow restrictor (350).

## Patentansprüche

1. Aerosolerzeugungssystem, aufweisend:
eine Vorrichtung (10), umfassend eine Energieversorgung (16) und eine elektrische Heizvorrichtung, wobei die elektrische Heizvorrichtung (22) mit der Energieversorgung (16) verbunden ist; und
eine Substratpatrone (20), enthaltend aerosolbildendes Substrat in Form eines thermoreversiblen Gels, das bei Raumtemperatur fest ist;
wobei die Substratpatrone (20) zum Einsetzen in oder Verbinden mit der Vorrichtung vor dem Gebrauch und zum Entfernen oder Trennen von der Vorrichtung (10) nach dem Gebrauch ausgelegt ist.

2. Aerosolerzeugungssystem nach Anspruch 1, wobei die elektrische Heizvorrichtung (22) nicht mit dem aerosolbildenden Substrat in Kontakt ist.

3. Aerosolerzeugungssystem nach Anspruch 1 oder 2, wobei wenigstens eine Wand der Substratpatrone (20) zwischen der elektrischen Heizvorrichtung (22) und dem aerosolbildenden Substrat vorgesehen ist.

4. Aerosolerzeugungssystem nach einem beliebigen vorhergehenden Anspruch, wobei die elektrische Heizvorrichtung (22) eine Widerstandsheizspur in oder auf einem starren Substratmaterial aufweist.

5. Aerosolerzeugungssystem nach einem beliebigen vorhergehenden Anspruch, wobei die elektrische Heizvorrichtung (22) in einem Schlitz (34) in der Patrone aufgenommen wird.

6. Aerosolerzeugungssystem nach Anspruch 5, wobei der Schlitz (34) ein Blindschlitz ist.

7. Aerosolerzeugungssystem nach einem beliebigen vorhergehenden Anspruch, wobei wenigstens eine Wand der Patrone (20) in thermischem Kontakt mit der Heizvorrichtung (22) steht.

8. Aerosolerzeugungssystem nach einem beliebigen vorhergehenden Anspruch, wobei die Substratpatrone (20) wenigstens eine flüssigkeits- und dampfundurchlässige Außenwand aufweist, die einen Blindhohlraum (34) definiert, wobei das aerosolbildende Substrat in dem Blindhohlraum (34) enthalten ist.

9. Aerosolerzeugungssystem nach Anspruch 8, wobei der Blindhohlraum (34) durch ein zerbrechliches, entfernbares oder dampfdurchlässiges Dichtungselement (50) abgedichtet ist.

10. Aerosolerzeugungssystem nach einem beliebigen vorhergehenden Anspruch, wobei die Vorrichtung (10) einen von der Patrone (20) getrennten Mundstückabschnitt (14) aufweist.

11. Aerosolerzeugungssystem nach einem beliebigen vorhergehenden Anspruch, wobei die Substratpatrone (20) eine erste Kammer (30) und eine von der ersten Kammer (30) getrennte zweite Kammer (32) aufweist.

12. Aerosolerzeugungssystem nach Anspruch 11, wobei wenigstens ein Abschnitt der elektrischen Heizvorrichtung (22) zwischen der ersten (30) und der zweiten (32) Kammer angeordnet ist.

13. Aerosolerzeugungssystem nach einem beliebigen vorhergehenden Anspruch, wobei das Gel eine Nikotinquelle oder ein Tabakprodukt aufweist.

14. Patrone für ein Aerosolerzeugungssystem, wobei das Aerosolerzeugungssystem eine Heizvorrichtung (22) aufweist, wobei die Patrone umfasst:
eine Substratpatrone (330), enthaltend ein aerosolbildendes Substrat in Form eines thermoreversiblen Gels, das bei Raumtemperatur fest ist, wobei die Patrone zur lösbaren Verbindung mit einem Körper des Aerosolerzeugungssystems oder zur Aufnahme in diesem ausgelegt ist und wobei die Patrone einen Schlitz (34) aufweist, der zur Aufnahme der Heizvorrichtung (22) ausgelegt ist.

15. Patrone nach Anspruch 14, wobei die Substratpatrone wenigstens eine flüssigkeits- und dampfundurchlässige Außenwand aufweist, die einen Blindhohlraum (30, 32) definiert, wobei das aerosolbildende Substrat in dem Blindhohlraum (30, 32) enthalten ist.

16. Patrone nach Anspruch 14 oder 15, wobei die Patrone ein Mundstückrohr (300) aufweist, wobei die Substratpatrone (330) in dem Mundstückrohr (300) gehalten wird und wobei das Mundstückrohr (300) ein Mundende zum Einführen in den Mund eines Benutzers aufweist.

17. Patrone nach Anspruch 16, wobei das Mundstückrohr (300) einen Luftstrombegrenzer (350) aufweist.

## Revendications

1. Système de génération d'aérosol, comprenant :
un dispositif (10) comprenant une alimentation électrique (16) et un dispositif de chauffage électrique, le dispositif de chauffage électrique (22) étant connecté à l'alimentation électrique (16) ; et
une cartouche de substrat (20) contenant un substrat formant aérosol sous la forme d'un gel thermoréversible qui est solide à température ambiante ;
dans lequel la cartouche de substrat (20) est configurée pour être insérée dans le dispositif ou connectée à celui-ci avant utilisation et retirée ou déconnectée du dispositif (10) après utilisation.

2. Système de génération d'aérosol selon la revendication 1, dans lequel le dispositif de chauffage électrique (22) n'est pas en contact avec le substrat formant aérosol.

3. Système de génération d'aérosol selon la revendication 1 ou 2, dans lequel au moins une paroi de la cartouche de substrat (20) est prévue entre le dispositif de chauffage électrique (22) et le substrat formant aérosol.

4. Système de génération d'aérosol selon l'une quelconque des revendications précédentes, dans lequel le dispositif de chauffage électrique (22) comprend un piste chauffante résistive dans une matière de substrat rigide ou sur celle-ci.

5. Système de génération d'aérosol selon l'une quelconque des revendications précédentes, dans lequel le dispositif de chauffage électrique (22) est reçu dans une fente (34) dans la cartouche.

6. Système de génération d'aérosol selon la revendication 5, dans lequel la fente (34) est une fente aveugle.

7. Système de génération d'aérosol selon l'une quelconque des revendications précédentes, dans lequel au moins une paroi de la cartouche (20) est en contact thermique avec le dispositif de chauffage (22).

8. Système de génération d'aérosol selon l'une quelconque des revendications précédentes, dans lequel la cartouche de substrat (20) comprend au moins une paroi externe imperméable aux liquides et à la vapeur définissant une cavité aveugle (34), dans lequel le substrat formant aérosol est contenu dans la cavité aveugle (34).

9. Système de génération d'aérosol selon la revendication 8, dans lequel la cavité aveugle (34) est fermée hermétiquement par un élément de fermeture hermétique (50) cassable, amovible ou perméable à la vapeur.

10. Système de génération d'aérosol selon l'une quelconque des revendications précédentes, dans lequel le dispositif (10) comprend une partie d'embout buccal (14) séparée de la cartouche (20).

11. Système de génération d'aérosol selon l'une quelconque des revendications précédentes, dans lequel la cartouche de substrat (20) comprend une première chambre (30) et une deuxième chambre (32) séparée de la première chambre (30).

12. Système de génération d'aérosol selon la revendication 11, dans lequel au moins une partie du dispositif de chauffage électrique (22) est positionnée entre les première (30) et deuxième (32) chambres.

13. Système de génération d'aérosol selon l'une quelconque des revendications précédentes, dans lequel le gel comprend une source de nicotine ou un produit du tabac.

14. Cartouche pour un système de génération d'aérosol, le système de génération d'aérosol comprenant un dispositif de chauffage (22), la cartouche comprenant :
une cartouche de substrat (330) contenant un substrat formant aérosol sous la forme d'un gel thermoréversible qui est solide à température ambiante, dans laquelle la cartouche est configurée pour se connecter de manière amovible à un corps du système de génération d'aérosol ou être reçue dans celui-ci et dans laquelle la cartouche comprend une fente (34) configurée pour recevoir le dispositif de chauffage (22).

15. Cartouche selon la revendication 14, dans laquelle la cartouche de substrat comprend au moins une paroi externe imperméable aux liquides et imperméable aux vapeurs définissant une cavité aveugle (30, 32), dans laquelle le substrat formant aérosol est contenu dans la cavité aveugle (30, 32).

16. Cartouche selon la revendication 14 ou 15, dans laquelle la cartouche comprend un tube d'embout buccal (300), dans laquelle la cartouche de substrat (330) est maintenue dans le tube d'embout buccal (300), et dans laquelle le tube d'embout buccal (300) a une extrémité buccale destinée à l'insertion dans la bouche d'un utilisateur.

17. Cartouche selon la revendication 16, dans laquelle le tube d'embout buccal (300) comprend un réducteur d'écoulement d'air (350).
